# EUROPÄISCHE PATENTSCHRIFT

(11) **EP 3 747 855 B1**
(45) Veröffentlichungstag und Bekanntmachung des Hinweises auf die Patenterteilung: **10.01.2024**
(21) Anmeldenummer: 19178117.8
(22) Anmeldetag: 04.06.2019
(51) Int. Cl.: C07C 29/141, C07C 31/20, C07C 45/75, C07C 47/19, B01J 25/00, B01J 25/02, B01J 23/75, B01J 23/89

(54) **VERFAHREN ZUR KONTINUIERLICHEN HERSTELLUNG VON DIOLEN AUS ALDEHYDEN MITTELS RANEY-COBALT KATALYSE**
METHOD FOR THE CONTINUOUS PREPARATION OF DIOLS FROM ALDEHYDES USING RANEY-COBALT CATALYSIS
PROCÉDÉ DE FABRICATION CONTINUE DE DIOLES À PARTIR DES ALDÉHYDES AU MOYEN DU CATALYSEUR COBALT DE RANEY

(43) Veröffentlichungstag der Anmeldung: 09.12.2020
(73) Patentinhaber: OQ Chemicals GmbH, 40789 Monheim am Rhein (DE)
(72) Erfinder: Reimer, Joachim, 68165 Mannheim (DE); Schalapski, Kurt, 46147 Oberhausen (DE); Rahe, Jan Henry, 45701 Herten (DE); Arnold, Jörg, 46535 Dinslaken (DE); Musenbrock, Marcel, 40629 Düsseldorf (DE)
(74) Vertreter: Michalski Hüttermann & Partner Patentanwälte mbB

(56) Entgegenhaltungen:
- WO-A2-2008/151102
- WO-A2-2010/000382
- DE-A1- 1 804 984

## Beschreibung

Die vorliegende Erfindung betrifft ein Verfahren zur kontinuierlichen Herstellung von C4-C10 Diolen aus C3-C9 Aldehyden umfassend die Verfahrensschritte: a) basenkatalysierte Addition von Formaldehyd an C3-C9 Aldehyde unter Erhalt der entsprechenden Hydroxyaldehyde, und b) anschließende Hydrierung der Hydroxyaldehyde zu den entsprechenden Diolen, wobei die Hydrierung der Hydroxyaldehyde ohne Aufarbeitung der Reaktionsmischung aus dem Verfahrensschritt a) kontinuierlich in flüssiger Phase an einem Raney^{™}-Cobalt-Katalysator in Gegenwart von Wasserstoff durchgeführt wird.

Die gezielte Funktionalisierung organischer Moleküle im großtechnischen Maßstab ist für die chemische Industrie auch heutzutage noch eine große Herausforderung. Dies liegt darin begründet, dass neben der Anwendung im Prinzip bekannter Reaktionsmechanismen im großtechnischen Umfeld weitere, komplexe Abhängigkeiten zu anderen Prozessparametern bestehen, welche im Endergebnis über die Wirtschaftlichkeit und die Wettbewerbsfähigkeit des zu nutzenden Verfahrens entscheiden. So spielen in der industriellen Praxis neben fundamentalen Größen wie Umsatz und Selektivität auch energetische, Sicherheits-, Umwelt- und Prozesszeiten-Aspekte eine wesentliche Rolle. Diese Querabhängigkeiten können dazu führen, dass vielversprechende Labor-Syntheserouten unter kontinuierlichen Bedingungen im Großmaßstab unwirtschaftlich sind und Alternativlösungen, trotz a priori ungünstigerer Randbedingungen, eine bessere Wahl darstellen.

Mehrwertige Alkohole oder Polyole besitzen als Kondensationskomponente zum Aufbau von Polyestern oder Polyurethanen, Kunstharzlacken, Schmiermitteln und Weichmachern eine erhebliche wirtschaftliche Bedeutung. Ein wichtiger Vertreter dieser Klasse ist Neopentylglykol (NPG, 2,2-Dimethylpropandiol-(1,3)), welches großtechnisch über eine gemischte Aldoladdition zwischen Formaldehyd und iso-Butanal erhalten werden kann. Bei der Aldoladdition bildet sich zunächst ein Hydroxyaldehyd, welches üblicherweise isoliert und in einem separaten Verfahrensschritt zum mehrwertigen Alkohol reduziert werden muss.

Für die Umsetzung von Aldehyden zu Alkoholen wird in der wissenschaftlichen und der Patentliteratur eine Vielzahl von Verfahren angeführt, welche eine metallkatalysierte Umsetzung in Gegenwart von Wasserstoff vorschlagen. Neben der Gemeinsamkeit der Umsetzung der spezifischen funktionalen Gruppen, variieren die Verfahren aber im erheblichen Maße. So finden sich Batch- vs. kontinuierliche Verfahrensführungen, Gas- vs. Flüssigphasenreaktionen, die Hydrierung der isolierten Aldehyde oder die Umsetzung in einer komplexeren Reaktionsumgebung unter der Anwesenheit weiterer Substanzen. Diese Unterschiede in den Prozessen haben dazu geführt, dass sich als Funktion des spezifischen Verfahrensweges, sich jeweils andere großtechnische Randbedingungen als vorteilhaft herausgestellt haben.

So beschreibt beispielsweise die WO 2014/067602 A1 ein kontinuierliches Verfahren zur Herstellung von Neopentylglykol durch Addition von iso-Butanal und Formaldehyd in Gegenwart eines tertiären Alkylamins als Katalysator zum Hydroxypivalaldehyd mit nachfolgender Gasphasenhydrierung an einem barium- und mangandotierten Kupferchromit-Katalysator bei einer Temperatur von 125 bis 180°C und bei einem Überdruck von 30 bis 120 kPa.

Eine andere Möglichkeit in der Flüssigphase offenbart beispielsweise die WO 2014/067600 A1. Dieses Patentdokument beschreibt ein Verfahren zur Herstellung von Neopentylglykol durch Addition von iso-Butanal und Formaldehyd in Gegenwart eines tertiären Alkylamins als Katalysator zum Hydroxypivalaldehyd mit nachfolgender Flüssigphasenhydrierung an einem barium- und mangandotierten Kupferchromit-Katalysator bei einer Temperatur von 80 bis 220°C und bei einem Druck von 2 bis 18 MPa.

Ein anderer Weg in der Flüssigphase wird beispielsweise in der WO 2010/000382 A2 offenbart, welche ein Verfahren zur Herstellung von Neopentylglykol durch Addition von iso-Butanal und Formaldehyd in Gegenwart eines tertiären Alkylamins als Katalysator zum Hydroxypivalaldehyd mit nachfolgender Flüssigphasenhydrierung an einem Nickelkatalysator bei einer Temperatur von 80 bis 180°C und bei einem Druck von 6 bis 18 MPa in Gegenwart eines aliphatischen Alkohols und in Gegenwart von Wasser betrifft.

Eine weitere Verfahrensvariante zum Erhalt von Alkoholen ist in der WO 95/32171 angegeben. Dieses Dokument offenbart ein Verfahren zur Herstellung von Alkoholen durch die katalytische Hydrierung der entsprechenden Carbonylverbindungen bei erhöhter Temperatur und bei erhöhtem Druck in flüssiger Phase, wobei man einen Katalysator verwendet, der Kupfer auf einem SiO₂-haltigen Trägermaterial in An- oder Abwesenheit eines oder mehrerer der Elemente Magnesium, Barium, Zink oder Chrom enthält.

Zusätzlich offenbart die WO2008/151102 A2 ein Verfahren zum Hydrieren eines Aldehyds. In einem Aspekt betrifft die Erfindung ein Verfahren zum Hydrieren eines Aldehyds mit einem Katalysator, der ein Metall der Gruppe VIII umfasst, wobei der Katalysator mit Kohlenmonoxid komplexiert ist, bei einer Temperatur von mindestens 120°C. In einem anderen Aspekt betrifft die Erfindung ein Verfahren zum Hydrieren eines Aldehyds durch Inkontaktbringen einer Beschickung, die den Aldehyd umfasst, mit einem Metallkatalysator der Gruppe VIII und Wasserstoff in Gegenwart von Kohlenmonoxid bei einer Temperatur von höchstens 90°C und anschließendes Inkontaktbringen der Beschickung und Katalysator mit Wasserstoff bei einer Temperatur von mindestens 120°C.

Trotz der schon bekannten Verfahren zur Herstellung von Alkoholen aus Aldehyden besteht weiterhin ein gesteigertes Interesse an industriellen Verfahren, welche in der Lage sind, unter hohen Durchsätzen und sehr effizient, auch komplexe Reaktionsgemische umzusetzen.

Es ist daher die Aufgabe der vorliegenden Erfindung ein integrales Verfahren bereitzustellen, welches die Nachteile der bekannten Verfahren, zumindest in Teilen, überwindet und eine kontinuierliche Umsetzung von Aldehyden zu Hydroxyaldehyden und, ohne Aufarbeitung dieser Reaktionslösung, eine anschließende Hydrierung der Hydroxyaldehyde zu den entsprechenden Diolen erlaubt.

Erfindungsgemäß wird daher ein Verfahren zur 2-stufigen Synthese von Diolen gemäß Anspruch 1 vorgeschlagen. Vorteilhafte Weiterbildungen des Verfahrens sind in den Unteransprüchen angegeben. Sie können beliebig kombiniert werden, sofern sich aus dem Kontext nicht eindeutig das Gegenteil ergibt.

Erfindungsgemäß umfasst das Verfahren zur kontinuierlichen Herstellung von C4-C10 Diolen aus C3-C9 Aldehyden die Verfahrensschritte:
a. basenkatalysierte Addition von Formaldehyd an C3-C9 Aldehyde unter Erhalt der entsprechenden Hydroxyaldehyde, und
b. anschließende Hydrierung der Hydroxyaldehyde zu den entsprechenden Diolen, wobei die Hydrierung der Hydroxyaldehyde ohne Aufarbeitung der Reaktionsmischung aus dem Verfahrensschritt a) kontinuierlich in flüssiger Phase an einem Raney-Cobalt-Katalysator in Gegenwart von Wasserstoff durchgeführt wird.

Es wurde gefunden, dass obige Prozessführung, basierend auf einem Hydrierungsschritt in komplexer Reaktionsumgebung unter Raney^{™}-Cobalt-Katalyse, mehrere, unerwartete Vorteile für die Reaktionsmischung und damit für den gesamten Syntheseweg aufweist. Einige der Vorteile ergeben sich wie folgt:
- Es lassen sich die Hydroxyaldehyde in der komplexen Reaktionsmischung der Aldolisierung umsetzen, sodass eine komplizierte und energieintensive Aufarbeitung der Reaktionsmischung vor der Hydrierung entfallen kann.
- Es lassen sich in breiten Prozessparameterbereichen stabile Prozessbedingungen mit hohen Umsätzen und Selektivitäten für beide Reaktionsschritte erreichen.
- Hohe Umsätze und Selektivitäten werden auch unter hohen Katalysatorbelastungen erreicht.
- Der Katalysator zeigt in der Reaktionsumgebung eine ausgezeichnete Stabilität und neigt insbesondere nicht zu mechanischer oder chemischer Zersetzung. Dies garantiert lange Standzeiten und vermeidet/verbessert eine aufwendige Abtrennung der Metalle aus dem Produkt.
- Es lässt sich der Gehalt unerwünschter Nebenprodukte aus der Aldolisierung, wie beispielsweise Ameisensäure oder deren Addukte, in der Reaktionsmischung des Verfahrensschrittes b) reduzieren.
- Es erfolgt nur ein geringer Abbau der Basen des Reaktionsschrittes a) (beispielsweise Tri-n-Propylamin (TPA)) in der Hydrierung, welches zu weniger störenden Nebenprodukten im Produkt führt und insgesamt die Rückführung des Amins in Prozessschritt a) verbessert.
- Die Hydrierung ist hoch effizient und kann sogar in einem niedrigen Temperaturbereich gefahren werden, welches die Bildung unerwünschter Nebenprodukten insgesamt verringert.

Die Kombination der oben erwähnten Verfahrensschritte unter den geforderten Prozessbedingungen, führt also nicht nur zu einem hohen Umsatz, einer hohen Selektivität und einer hohen Energieeffizienz, sondern stellt auch sicher, dass der Prozess stabil, über einen langen Zeitraum ohne erhöhten Wartungsaufwand betrieben werden kann. Diese Vorteile ergeben in Summe ein sehr wirtschaftliches und umweltfreundliches Verfahren.

Das erfindungsgemäße Verfahren ist ein Verfahren zur kontinuierlichen Herstellung von C4-C10 Diolen aus C3-C9 Aldehyden. Unter einer kontinuierlichen Herstellung werden dabei Verfahrensführungen verstanden, in welchen als Funktion der Zeit, Edukte nicht nur einmal, sondern entweder kontinuierlich oder aber häufig innerhalb kurzer Zeitabstände, zum Reaktionsort hinzugegeben werden. Ebenso verhält es sich mit den Produkten, welche nicht nach einer vorgegebenen Zeitspanne in Summe, sondern in regelmäßigen Zeitabständen oder kontinuierlich dem Reaktionsort entzogen werden.

Als Edukte des Verfahrens dienen C3-C9 Aldehyde. Als Einsatzaldehyde kommen als aliphatische und aromatische Kohlenwasserstoffe mit 3 bis 9 C-Atomen und einer Aldehydgruppe (R-CHO) in Frage. Mögliche Einsatzaldehyde können beispielsweise aus der Gruppe der aliphatischen Aldehyde wie Propanal, Butanale, Pentanale, Hexanale, Heptanale, Octanale und Nonanale ausgesucht sein. Gerade diese Gruppe der eher niedermolekularen Aldehyde zeigen in der erfindungsgemäßen Umsetzung gleichbleibend gute Eigenschaften.

Die erfindungsgemäß einsetzbaren Aldehyde werden zu den entsprechenden C4-C10 Diolen umgesetzt. Dies bedeutet, dass mittels der erfindungsgemäßen Umsetzungen das C-Gerüst des Einsatzaldehydes um mindestens ein C-Atom verlängert, eine weitere Hydroxylgruppe ins Edukt eingeführt und die bestehende Aldehydgruppe zu einer Hydroxylgruppe umgesetzt wird. Bevorzugt über das Verfahren erhältliche Diole sind beispielsweise Neopentylglykol, Trimethylolpropan und höhere Homologa.

Das Verfahren umfasst in einem ersten Verfahrensschritt a) die basenkatalysierte Addition von Formaldehyd an C3-C9 Aldehyde unter Erhalt der entsprechenden Hydroxyaldehyde. Im folgenden Reaktionsschema ist die prinzipielle Umsetzung im Verfahrensschritt a) anhand einer Umsetzung von iso-Butanal dargestellt:

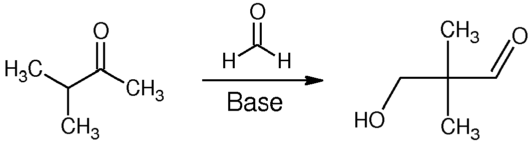

Durch die Umsetzung in diesem Schritt erhält man Hydroxypivalaldehyd als Zwischenprodukt. Als Basen eignen sich generell sowohl anorganische wie organische Basen. Bevorzugte anorganische Basen sind beispielsweise die Hydroxide der Alkali- wie auch der Erdalkalimetalle. Als organische Basen können tertiäre Alkylamine mit mehreren Trialkylaminfunktionen eingesetzt werden. Beispielsweise wird in Gegenwart von Trimethyl-, Triethyl-, Tri-n-propyl-, Tri-iso-propyl-, Methyl-diethyl-, Methyl-diisopropylamin, Tri-n-butylamin, Dimethyl-tert-butylamin oder N,N'-Tetramethylethylendiamin gearbeitet.

Die Aldehyde können im Verfahrensschritt a) im molaren Verhältnis mit Formaldehyd umgesetzt werden, jedoch ist es auch möglich, einen der beiden Reaktionspartner im Überschuss anzuwenden. Man kann Formaldehyd als wässrige Lösung einsetzen. Der Formaldehydgehalt kann üblicherweise von 20 bis 50 Gew.-% betragen. Es hat sich herausgestellt, dass der im erfindungsgemäßen Verfahren verwendete Katalysator eine überraschend hohe Resistenz gegenüber Formaldehyd besitzt. Daher können in der Aldoladditionsstufe Molverhältnisse von Formaldehyd zu Aldehyd von 1:1, im Allgemeinen bis zu 1,2:1, vorzugsweise bis 1,1:1 eingestellt werden.

Die Reaktion zwischen Aldehyd und Formaldehyd kann bei Temperaturen zwischen 20 und 110°C erfolgen. Zweckmäßigerweise arbeitet man bei 80 bis 95°C. Im Allgemeinen wird die Reaktion bei Normaldruck durchgeführt, jedoch kann man auch erhöhten Druck anwenden. Die als Katalysator verwendete Base kann in dem Reaktionsgemisch in einer Menge von 1 bis 20, vorzugsweise 2 bis 12 Mol.-%, bezogen auf das Aldehyd, enthalten sein.

Neben dem Wasser aus der wässrigen Formaldehydlösung und geringen Anteilen an Methanol, das ebenfalls in der wässrigen Formaldehydlösung enthalten sein kann, wird gegebenenfalls der Reaktionsmischung noch iso-Butanol als Verdünnungsmittel zugesetzt. Der iso-Butanolzusatz ist nicht zwingend erforderlich, falls jedoch iso-Butanol zugesetzt wird, liegt sein Gehalt in der Reaktionsmischung im Bereich von 15 bis 30 Gew.-%, vorzugsweise 15 bis 25 Gew.-%, bezogen auf den organischen Anteil im gesamten Reaktionsgemisch. Weitere Lösungs- und Verdünnungsmittel sind nicht erforderlich.

Die praktische Durchführung der Additionsreaktion erfolgt beispielsweise in einem Rührkessel, in einer Rührkesselkaskade oder in einem Reaktionsrohr, das zur besseren Durchmischung der Reaktanten mit Füllkörpern oder anderen Einbauten beschickt sein kann. Die Umsetzung verläuft exotherm, sie kann durch Erhitzen beschleunigt werden.

Das nach der Aldoladdition anfallende Rohgemisch wird ohne vorherige Trennung in seine Bestandteile oder Abtrennung einzelner Komponenten in Gegenwart des Raney^{™}-Cobalt-Katalysators katalytisch hydriert.

Im Verfahrensschritt b) erfolgt die Hydrierung der Hydroxyaldehyde zu den entsprechenden Diolen. Unter Fortsetzung der unter Verfahrensschritt a) gezeigten beispielhaften Umsetzung ergibt sich beispielsweise eine Umsetzung im Verfahrensschritt b) wie folgt:

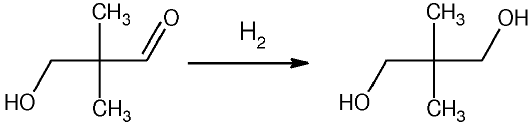

Man erhält in dieser Umsetzung Neopentylglykol als Reaktionsprodukt, welches aus der komplexen Reaktionsmischung durch die üblichen Aufreinigungsschritte und -Verfahren isoliert werden kann. Weitere bevorzugt herstellbaren Diole sind beispielsweise Neopentylglykol, Trimethylolpropan oder höhere Homologa.

Die Hydrierung der Hydroxyaldehyde im Verfahrensschritt b) erfolgt ohne Aufarbeitung der Reaktionsmischung aus dem Verfahrensschritt a). Dies bedeutet, dass die Hydrierung im Wesentlichen an derselben Reaktionsmischung durchgeführt, wie sie aus dem Verfahrensschritt a) erhalten wurde. Eine solche Umsetzung erfolgt im Wesentlichen nicht an derselben Reaktionsmischung, und insofern nicht ohne Aufarbeitung, in den Fällen, in denen der Reaktionslösung eine oder mehrere Komponenten durch weitere verfahrenstechnische Operationen ganz oder teilweise gezielt entzogen werden. Dies beispielsweise durch übliche chemische Trennoperationen wie eine Destillation. Eine Reaktionslösung wird beispielsweise ohne Aufarbeitung eingesetzt, wenn die Konzentrationen der in ihr enthaltenen Komponenten zwischen Ende des Verfahrensschrittes a) und dem Start des Verfahrensschrittes b) weniger als 5 mol%, bevorzugt weniger als 2,5 mol%, des Weiteren bevorzugt weniger als 1,5 mol% pro Komponente variieren. Eine Reaktionslösung wird im Wesentlichen ohne Aufarbeitung eingesetzt, wenn sich eventuelle Konzentrationsänderungen in der Lösung ohne bewussten äußeren Eingriff, beispielsweise durch eine Druck- oder Temperaturänderung, ergeben.

Der Verfahrensschritt b), also die Hydrierung, erfolgt kontinuierlich in flüssiger Phase, beispielsweise an fest angeordneten Katalysatoren nach Riesel- oder Sumpffahrweise sowie nach der Suspensionshydrierung. Dies bedeutet insbesondere, dass die Hydrierung nicht im Rahmen einer reinen Gasphasenreaktion erfolgt. Die Umsetzung der Aldehyd- zur Alkoholgruppe kann beispielsweise durch den Einsatz gasförmigen Wasserstoffs erfolgen, welcher durch die bei den Reaktionsbedingungen flüssige Reaktionsmischung des oder der weiteren Edukte geführt wird. Die Hydrierung erfolgt unter kontinuierlicher Zuführung mindestens eines Eduktes und kontinuierlichem Entfernen mindestens eines Produktes aus dem Reaktionsraum.

Vorzugsweise erfolgt die Hydrierung in Gegenwart eines aliphatischen Alkohols, der mit dem Aldolisierungsrohprodukt mischbar ist. Als geeignete aliphatische Alkohole haben sich lineare oder verzweigte Alkohole mit 1 bis 5 Kohlenstoffatomen beispielsweise Methanol, Ethanol, n-Propanol, iso-Propanol, n-Butanol, iso-Butanol, Neopentylglykol oder Mischungen davon erwiesen. Besonders zweckmäßig verwendet man iso-Butanol, da Restmengen von iso-Butanal zu iso-Butanol hydriert werden. Falls man iso-Butanol bereits in der Aldoladditionsstufe als Verdünnungsmittel einsetzt, liegt in der Hydrierstufe schon ein Lösungsmittel vor. Geringe Mengen an Methanol, die über die wässrige Formaldehydlösung eingetragen werden, sind ebenfalls zugegen. Der Anteil des aliphatischen Alkohols als organisches Lösungs- oder Verdünnungsmittel kann in dieser Ausgestaltung der Erfindung 15 bis 27 Gew.-%, vorzugsweise 15 bis 23 Gew.-%, bezogen auf den organischen Anteil in dem Einsatzgemisch betragen. Durch den Zusatz des Verdünnungs- oder Lösungsmittels wird eine bevorzugte Löslichkeit des Hydroxypivalaldehyds in der flüssigen Phase während der Hydrierstufe sichergestellt sowie das Ausfallen von Hydroxypivalaldehyd verhindert und die Homogenität der flüssigen Phase gewährleistet. Bei zu hohen Alkoholgehalten wird das Reaktorvolumen unnötig belegt.

Der Verfahrensschritt b) wird erfindungsgemäß an einem Raney^{™}-Cobalt-Katalysator durchgeführt. Raney^{™}-Cobalt-Katalysatoren werden üblicherweise durch Behandeln eines Metalllegierungspulvers mit einem Alkali hergestellt, wobei die Zusammensetzung der Metalllegierung, bezogen auf das Gewicht ca. 20% bis 60% Cobalt und ggf. weitere Metalle wie Eisen, Nickel, Chrom, Rhodium, Ruthenium, Osmium, Iridium, Platin, Palladium und Mischungen der Metalle dieser Gruppe aufweist. Der Rest der Legierungszusammensetzung ist ein Metall, löslich in Alkali. Die alkalilöslichen Metalle schließen ein Aluminium, Zink, Magnesium und Silizium. Aluminium ist das bevorzugte alkalilösliche Metall. Die Legierung aus der der Katalysator hergestellt ist, kann durch übliche metallurgische Verfahren hergestellt werden, die Legierungsschmelzlinge herstellen. Zum Erhalten der Legierung in der gewünschten Pulverform wird der Schmelzling zerkleinert und gemahlen. Das Legierungspulver wird zum aktiven Katalysator durch Behandlung mit einer wässrigen alkalischen Lösung, vorzugsweise Natriumhydroxid, umgewandelt. Diese Lösung wäscht die Mehrheit des Aluminiums oder anderes alkalilösliches Metall unter Ergeben des aktiven Raney^{™} Metallkatalysators aus. Der Cobaltgehalt der aktiven Katalysatoren auf Trockenbasis kann ca. 25% bis etwa 80% betragen. Der Rest der Katalysatorzusammensetzung ist eine Funktion des Vorliegens weiterer Metalle als Promotoren und der Gründlichkeit des Auswaschverfahrens. Allgemein verbleibt auch eine geringe Menge des alkalilöslichen Metalls, beispielsweise Aluminium, im Katalysator. Die alkalilöslichen Metallreste können als Oxide vorhanden sein.

Der Cobalt-Hydrierungskatalysator kann ganz allgemein zu dem katalytischen wirksamen Hauptmetall Dotierungsmetalle umfassen, ausgewählt unter den Elementen der Gruppen Ib, IIb, IVb, VIb, VIIb und VIII des Periodensystems der Elemente, sowie Aluminium, das insbesondere in den Raney^{™}-Metallen vorliegt.

Die Hydrierung des rohen Hydroxypivalaldehyds kann bei einer Temperatur von 60 bis 220°C, vorzugsweise von 60 bis 180°C und insbesondere von 70 bis 160°C, in der flüssigen Phase in Gegenwart von Raney^{™}-Cobalt-Katalysatoren durchgeführt werden. Temperaturen von 60-140°C können bevorzugt sein, da die Temperatur der Reaktionsmischung aus dem Verfahrensschritt a) ebenfalls bevorzugt in diesem Bereich liegen kann. Letzteres kann aufwendige weitere Temperierungen zwischen den Verfahrensschritten vermeiden. Der Reaktionsdruck beträgt bevorzugt 2 bis 150 MPa, vorzugsweise 60 bis 120 MPa. Besonders bewährt haben sich eine Reaktionstemperatur von 70 bis 160°C und ein Reaktionsdruck von 60 bis 120 MPa. Bei niedrigeren Reaktionsdrücken wird keine zufriedenstellende Hydrierung beispielsweise von Hydroxypivalaldehyd mehr beobachtet.

Bei der kontinuierlichen Festbettfahrweise hat sich eine Katalysatorbelastung V/Vh, ausgedrückt in Durchsatzvolumen pro Katalysatorvolumen und Zeit, von 0,2 bis 4,0 h-¹, vorzugsweise 0,3 bis 1,5 h⁻¹, als zweckmäßig erwiesen.

Vorzugsweise wird die Hydrierung kontinuierlich in flüssiger Phase in einem Rohrreaktor an fest angeordneten Katalysatoren durchgeführt. Unter einem Rohrreaktor ist auch ein Bündel von mehreren eng parallel geschalteten Rohren zu verstehen. Die eingesetzten Rohrreaktoren können ebenfalls Füllkörper oder Einbauten enthalten, beispielsweise Raschigringe, Sättel, Prallringe, Filterplatten oder Kolonnenböden, sowie gegebenenfalls Rührvorrichtungen oder Vorrichtungen zum Abführen der Reaktionswärme. In einer besonders bevorzugten Ausgestaltung erfolgt die Hydrierung von Hydroxypivalaldehyd in einem Rohrreaktor am Festbett jedoch ohne Einbauten und ohne Rührvorrichtungen.

In einer bevorzugten Ausführungsform des Verfahrens kann der Raney-Cobalt-Katalysator in der Form eines Festbettes vorliegen. Unter einem Festbettreaktor versteht man eine Reaktorform in dem ein oder mehrere Fluide und/oder Gase durch eine feste Schüttung oder Packung strömen. Insbesondere der Einsatz des Katalysators als Festbett hat sich als im erfindungsgemäßen Verfahren als besonders vorteilhaft erwiesen. Die Festbettschüttung verhindert eine zu große mechanische Belastung der einzelnen Katalysatorpartikel und kann zu einer größeren Wasserstoffaustauschfläche beitragen. Dies kann zu höheren Umsätzen und zu einer geringen Belastung des Endproduktes mit unerwünschten Metallspuren beitragen.

Der Verfahrensschritt b), die Hydrierung, wird in Gegenwart von Wasserstoff durchgeführt. Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten. Zweckmäßigerweise wird die Hydrierung durch Aufpressen von Wasserstoff in den Reaktionsbehälter durchgeführt, wobei der Wasserstoffdruck generell zwischen 0,1 und 200 MPa betragen kann. Das Wasserstoffgas kann dabei durch den Fachmann bekannte technische Maßnahmen auf oder in den Reaktor gegeben werden, wobei es vorteilhaft sein kann, dass der Wasserstoff durch die flüssige Reaktionsmischung geleitet wird.

In einer weiteren Ausgestaltung des Verfahrens kann im Verfahrensschritt a) iso-Butanal mit wässriger Formaldehydlösung zu Hydroxypivalaldehyd umgesetzt und im Verfahrensschritt b) das Hydroxypivalaldehyd zu Neopentylglykol hydriert werden. Überraschenderweise hat sich gezeigt, dass sich mit dem erfindungsgemäßen Verfahren insbesondere oben genannte Umsetzungen unter Nutzung eines Raney^{™}-Cobalt-Katalysators besonders effizient bewerkstelligen lassen. Es lassen sich selbst bei sehr hohen Durchsätzen hohe Umsätze und hohe Selektivitäten erreichen, wobei, ohne durch die Theorie gebunden zu sein, dies zum Teil auf eine bevorzugte Ausrichtung der Edukte auf dem Raney^{™}-Cobalt-Katalysator und auf die chemische Reaktionsumgebung des Verfahrensschrittes a) zurückgeführt wird.

In einer bevorzugten Verfahrensvariante kann die Base im Verfahrensschritt a) aus der Gruppe bestehend aus Trimethylamin, Triethylamin, Tri-n-propylamin oder Tri-n-butylamin oder Mischungen mindestens zweier Bestandteile daraus ausgesucht sein. Insbesondere der Einsatz organischer Basen als Katalysator kann nicht nur zum Erhalt eines möglichst effektiven ersten Verfahrensschrittes a) beitragen. Es hat sich zudem auch für den Verfahrensschritt b) als vorteilhaft herausgestellt, wenn dieser in Gegenwart der oben genannten tertiären Amine an einem Raney^{™}-Cobalt-Katalysator durchgeführt wird. Diese Basen beeinflussen die Hydrierung nicht negativ und werden durch das gewählte Katalysatorsystem auch nicht im nennenswerten Umsatz umgewandelt, sodass diese nach Aufarbeitung wieder in den Verfahrensschritt a) zurückgegeben werden können.

Vorteilhaft ist auch, dass sich bei thermischer Abtrennung der Base durch den Energieeintrag vermehrt Hochsieder (insbesondere Hydroxypivalinsäure-Neopentylglykolester, HPN) bilden würden, die unter den milden Hydrierbedingungen nur zu geringen Anteilen wieder in Wertprodukt gespalten werden können.

Innerhalb einer weiteren Ausgestaltung des Verfahrens kann der Raney^{™}-Cobalt-Katalysator weitere Metalle ausgewählt aus der Gruppe bestehend aus Chrom, Molybdän, Eisen, Nickel, Kupfer, Ruthenium, Palladium, Platin, Mangan, Rhodium oder Mischungen mindestens zweier Bestandteile daraus umfassen. Neben der Umsetzung an einem reinen Raney^{™}-Cobalt-Katalysator hat es sich bezüglich des Umsatzes und der Prozessstabilität als günstig herausgestellt, dass neben der Raney^{™}-Legierung mit Cobalt als Bestandteil noch weitere Metalle im Katalysator vorliegen. Dies kann die Standzeiten des Katalysators verlängern und eine stabile Prozessführung über einen großen Prozess-Parameterbereich ermöglichen. Innerhalb einer bevorzugten Ausführungsform des Verfahrens kann der Raney-Cobalt-Katalysator neben Cobalt auch die Metalle Chrom und Nickel in einem Gewichtsanteil von größer oder gleich 0,05% und kleiner oder gleich 10% umfassen. Gerade der Zusatz von Chrom und Nickel kann in Rahmen eines kontinuierlichen Verfahrens zu einem effizienten Fahren auch unter höherer Katalysatorbelastung beitragen. Es können größere Eduktmengen pro Zeiteinheit umgesetzt werden, sodass die Wirtschaftlichkeit des Verfahrens gesteigert werden kann.

In einer zusätzlichen Verfahrensvariante kann die Hydrierung im Verfahrensschritt b) bei einer Temperatur von größer oder gleich 70°C und kleiner oder gleich 160°C durchgeführt werden. Die gewählte Verfahrensführung und das gewählte Katalysatorsystem können dazu beitragen, dass über einen breiten Temperaturbereich stabile Umsetzungen mit nur einem geringen Anteil an unerwünschten Nebenprodukten erhalten werden können. Dies ist erstaunlich, da dies selbst in einem eher moderaten oder niedrigeren Temperaturbereich unter hohen Umsätzen möglich ist. Es ergeben sich somit sehr wirtschaftliche Umsetzungen, welche sich zudem dadurch auszeichnen, dass der Aufwand zur Aufreinigung der Endprodukte geringgehalten werden kann. Bevorzugt kann die Temperatur im Verfahrensschritt b) größer oder gleich 95°C und kleiner oder gleich 145°C betragen.

In einer weiteren bevorzugten Ausführungsform des Verfahrens kann die Katalysatorbelastung LHSV (liquid hourly space velocity) im Verfahrensschritt b) größer oder gleich 0,3 h⁻¹ und kleiner oder gleich 1,5 h⁻¹ betragen. Trotz des Vorliegens einer sehr komplexen Reaktionsmischung, aufweisend Komponenten des Verfahrensschrittes a), können mit dem hier eingesetzten Katalysatorsystem hohe Katalysatorbelastungen, gegeben in Form der Raumgeschwindigkeit, definiert als Volumenstrom Edukt pro Volumen Katalysatorschüttung, erreicht werden, ohne dass das Gebiet hoher Umsätze und Selektivitäten verlassen wird. Dies ist insbesondere für kontinuierliche Verfahren von Vorteil und kann zu einer verbesserten Wirtschaftlichkeit und Energieeffizienz des Verfahrens beitragen.

Im Rahmen einer bevorzugten Ausgestaltung des Verfahrens kann die Konzentration der Base im Verfahrensschritt b) größer oder gleich 3 Gew.-% und kleiner oder gleich 15 Gew.-% betragen. Es hat sich gezeigt, dass das verwendete Raney^{™}-Cobalt-Katalysatorsystem höchst tolerant gegenüber der im Verfahrensschritt a) eingesetzten Base ist, sodass auch ohne vorherige Abtrennung und mit hohen Basenkonzentrationen sehr effiziente Hydrierungen im Verfahrensschritt b) durchgeführt werden können. Der Katalysator laugt auch unter den hohen Belastungen nicht aus, sodass eine unerwünschte Metallfracht im Endprodukt vermieden wird. Insgesamt führt dies zu einem effizienteren Gesamtprozess, da die im Verfahrensschritt a) eingesetzte Basen-Katalysatormenge effektiv von den Bedingungen im Verfahrensschritt b) entkoppelt werden kann.

In einer bevorzugten Ausgestaltung des Verfahrens kann das molare Verhältnis von Wasserstoff zu Hydroxyaldehyd im Verfahrensschritt b) größer oder gleich 1:1 und kleiner oder gleich 100:1 betragen. Zum Erhalt möglichst hoher Raumgeschwindigkeiten haben sich oben genannte molare Wasserstoff: Edukt-Verhältnisse als besonders vorteilhaft erwiesen. Die durch das Raney^{™}-Cobalt-Katalysatorsystem bereitgestellte Kinetik erlaubt die Zufuhr relativ großer Wasserstoffmengen, wobei der Anteil an unerwünschten Nebenprodukten sehr niedrig gehalten werden kann. Derart lassen sich insgesamt hohe Produktmengen in sehr kurzen Zeiträumen bereitstellen.

In einer bevorzugten Ausführungsform des Verfahrens kann der Druck im Verfahrensschritt b) größer oder gleich 60 MPa und kleiner oder gleich 120 MPa betragen. Trotz der im Verfahrensschritt b) vorliegenden, komplexen Reaktionsmischung hat es sich als vorteilhaft gezeigt, die Hydrierung bei relativ hohen Drücken durchzuführen. Dies kann die Kinetik der Hydrierung am Raney^{™}-Cobalt-Katalysatorsystem positiv beeinflussen und insgesamt zu einer verbesserten Anlagenauslastung beitragen. Besonders hohe Umsätze und Selektivitäten lassen sich bevorzugt zudem zwischen größer oder gleich 80 MPa und kleiner oder gleich 100 MPa erhalten.

In einer weiteren bevorzugten Ausgestaltung des Verfahrens kann der Hydroxyaldehydgehalt zu Beginn des Verfahrensschritts b) größer oder gleich 15 Gew.-% und kleiner oder gleich 85 Gew.-% betragen. Mittels des erfindungsgemäßen Verfahrens lassen sich auch komplexe Produktmischungen mit hohen Anteilen an Hydroxyaldehyden sicher und unter Vermeidung zu hoher Nebenprodukt-Anteile umsetzen. Der Hydroxyaldehydgehalt kann dabei beispielsweise über HPLC-Verfahren oder Gaschromatographie bestimmt werden. Des Weiteren kann der Gehalt bevorzugt größer oder gleich 25 Gew.-% und kleiner oder gleich 70 Gew.-% betragen.

In einer weiteren Alternative des Verfahrens kann die flüssige Phase im Verfahrensschritt b) einen Wassergehalt von größer oder gleich 5 Gew.-% und kleiner oder gleich 70 Gew.-% aufweisen. Das erfindungsgemäße Verfahren hat sich auch gegenüber relativ hohen Wassermengen als besonders robust herausgestellt. So führen diese Wassermengen nicht zu einer Deaktivierung des Raney^{™}-Cobalt-Katalysators. Des Weiteren können diese Wassermengen sogar dazu beitragen, dass eine niedrigere Menge an unerwünschten Nebenprodukten bereitgestellt werden kann.

In einer weiteren Alternative des Verfahrens kann die Reaktionslösung in Verfahrensschritt b) Ester umfassen, wobei der Estergehalt zu Beginn des Verfahrensschrittes b) größer oder gleich 0 Gew.-% und kleiner oder gleich 20 Gew.-% beträgt. Ein wesentlicher Vorteil des hier verwendeten Raney^{™}-Cobalt-Katalysators besteht darin, dass selbst unerwünschte Nebenprodukte des ersten Verfahrensschrittes im zweiten Verfahrensschritt wieder in die gewünschten Produkte überführt werden können. Somit ist das erfindungsgemäße 2-stufige Verfahren nicht nur stabil gegenüber der Präsenz hoher Nebenproduktmengen, es kann zudem zu einer deutlichen Steigerung der Ausbeute beitragen, indem diese Nebenprodukte gezielt in Wertprodukte umgewandelt werden. Ester sind dabei generell organische Substanzen, welche eine Estergruppe aufweisen. Zu diesen Estern können beispielsweise Tishchenko-Ester gehören, welche durch eine Tishchenko-Reaktion der Hydroxyaldehyde mit sich selbst oder mit anderen Aldehyden gebildet werden. Beispielhaft für diese Verbindungen sind Neopentylglykol-mono-isobutyrat und Hydroxypivalinsäureneopentylglykol-ester (HPN).

In einer weiteren bevorzugten Ausgestaltung des Verfahrens kann die Reaktionslösung in Verfahrensschritt b) Ameisensäure, deren Salze und/oder Ameisensäure-Amin-Addukte umfassen, wobei deren Gehalt zu Beginn des Verfahrensschrittes b) größer oder gleich 0 Gew.-% und kleiner oder gleich 3 Gew.-% beträgt. Es hat sich zudem als vorteilhaft herausgestellt, dass die erfindungsgemäße Verfahrensführung mit dem erfindungsgemäß verwendeten Raney^{™}-Cobalt-Katalysator äußerst tolerant auch gegenüber hohen Ameisensäure-Belastungen ist. Die Katalysatorleistung wird auch durch hohe Frachten nicht beeinträchtigt und zusätzlich hat sich gezeigt, dass die Ameisensäure auch unter hohen Katalysatorbelastungen und bei niedrigen Temperaturen im Verfahrensschritt b) quantitativ umgesetzt wird. Insbesondere letzteres kann zu einer weitgehenden Vermeidung des unerwünschten Nebenproduktes NPG-mono-Formiat beitragen.

In einer bevorzugten Ausführungsform können die Aldehyde aus der Gruppe der C3-C7 Aldehyde, des Weiteren bevorzugt aus der Gruppe der C3-C5 Aldehyde ausgesucht sein. Gerade die Aldehyde mit einer geringeren C-Anzahl können im Rahmen des erfindungsgemäßen Verfahrens besonders effizient unter hohen Reaktorbelastungen umgesetzt werden.

### Beispiele

### Methoden

Die Konzentrationsbestimmungen der organischen Substanzen, wie beispielsweise HPA, NPG, NPG-mono-iso-Butyrate, der Säuren (Hydroxypivalinsäure, Isobuttersäure) und der Tishchenko-Ester erfolgte über GC-FID. Die Konzentrationsbestimmungen der Ameisensäure erfolgte nach Derivatisierung mit N,O-Bis(trimethylsilyl)trifluoroacetamid (BSTFA) ebenfalls über GC-FID. Die Methode zur Konzentrationsbestimmung erfolgte in Anlehnung an DIN 51405.

Die Konzentrationsbestimmung der Aminverbindungen, beispielsweise der TPA Abbauprodukte, Di-n-Propylamin und Methyl-di-n-Propylamin, erfolgte über GC-PND in Anlehnung an DIN 51405 mit Tributylamin als internen Standard.

Die Bestimmung metallischer Komponenten erfolgte über ICP-OES/ICP-MS in Anlehnung an DIN 51405.

### Katalysatoren

Für die Vergleichsversuche wurden insgesamt 4 Katalysatoren getestet. Zum einen ein Kieselguhr geträgerter Nickel-Katalysator mit einer Schüttdichte von ca. 0,8 - 1,0 g/cm³ und drei Raney^{™}-Katalysatoren mit einer Schüttdichte zwischen 1,6 und 1,9 g/cm³.

### I. Batchversuch

### Verfahrensschritt a): Herstellung einer "rohen" HPA-Mischung

Der Verfahrensschritt a), in welchem die basenkatalysierte Addition von Formaldehyd an die Aldehyde unter Erhalt der entsprechenden Hydroxyaldehyde erfolgte, wird durch Reaktion von Iso-Butanal mit 49 %-iger wässriger Formalinlösung und einer Katalysatorlösung, die 30 Gew.-% Tri-n-Propylamin in iso-Butanol enthält, durchgeführt. Die Reaktion wird dabei zweckmäßigerweise in einer kontinuierlichen Rührkesselkaskade mit 2 Rührkesseln mit einem Volumen von jeweils 0,55 Litern durchgeführt. Die Einsatzströme betragen dabei 0,448 l/h iso-Butanal, 0,260 l/h 49%-ige Formalinlösung und 0,172 l/h Katalysatorlösung. Die Temperatur beträgt dabei im ersten Reaktor ~97°C und im zweiten Reaktor 103°C. Die Einsatzströme werden mittels Pumpen kontinuierlich gefördert. Der erste Reaktor dient vornehmlich der Durchmischung und dem Start der Reaktion. Im zweiten Reaktor reagiert die Reaktionsmischung weiter bis zum gewünschten Umsatzgrad.

Eine typische Zusammensetzung eines Reaktionsgemisches ergibt sich wie folgt (Angaben in GC-FI% (wasserfrei) und Gew.-% für Wasser):

| | |
|---|---|
| Ameisensäure (Silylierung) | 0,55 |
| Isobuttersäure (Silylierung) | 0,01 |
| Hydroxypivalinsäure (Silylierung) | 0,04 |
| Isobutanal | 1,58 |
| Methanol | 0,12 |
| TPA | 8,81 |
| Isobutanol | 19,46 |
| HPA | 59,81 |
| NPG-mono-iso-Butyrat | 0,61 |
| NPG | 1,92 |
| HPN | 3,96 |
| Andere Nebenkomponenten | 3,73 |
| Wasser (Karl-Fischer-Titration) | -20 |

### Verfahrensschritt b): Batchversuch Hydrierung

10 g Katalysator wurden in einen Edelstahlkorb gefüllt, welcher anschließend in einem Autoklaven mit 600 ml roher HPA-Lösung, welche aus dem Verfahrensschritt a) erhalten und ohne weitere Aufarbeitung eingesetzt wurde, eingebracht wurde. Der Autoklav wurde mit 80 kPa Wasserstoffgas beaufschlagt, auf 125°C aufgeheizt und für 12 Stunden ausreagiert. Die resultierenden Reaktionsmischungen wurden mittels GC untersucht.

| | **NPG in GC-Flächen-%** |
|---|---|
| Raney^{™}-Cobalt | 25,9 |
| Raney^{™}-Kupfer | 26,7 |
| Raney^{™}-Nickel | 24,8 |
| Nickel | 17,8 |

Der Batchversuch zeigt, dass die Raney^{™}-Katalysatoren eine vergleichbare quantitative Aktivität und somit eine vergleichbare Umsetzung der Zwischenstufe zum gewünschten NPG-Zielprodukt liefern. Die Umsetzungen liefern im Vergleich zu einem geträgerten Nickel-Katalysatorsystem signifikant höhere Umsätze. In den Batchversuchen ist bedingt durch die langen Verweilzeiten die Nebenkomponentenbildung sehr hoch, daher werden, verglichen mit dem HPA Gehalt im Einsatz, nur geringere Mengen NPG erhalten. Die Werte dienen somit nur zu einer ersten Orientierung.

### II. Kontinuierliche Herstellung

Die kontinuierlichen Hydrierungen wurden mittels eines Festbettreaktors mit 600 ml Katalysatorvolumen durchgeführt. Der Hydrierreaktor besteht hierbei aus einem Rohr mit 32 mm Innendurchmesser, welches von den Edukten (Reaktionsmischung aus Prozessschritt a) und Wasserstoff) von unten durchströmt wird. Das Rohr ist über einen Öl-Thermostaten mantelbeheizt und verfügt über ein zentrales Multi-Point-Thermoelement zur Temperaturmessung. Die angegebenen Temperaturen entsprechen jeweils dem Temperaturmaximum im Temperaturprofil über die Reaktorhöhe. Die Reaktionsmischung aus Verfahrensschritt a) wird kontinuierlich über eine Hochdruckpumpe zugeführt. Die angegebenen Katalysatorbelastungen/Durchsätze (LHSV) berechnen sich aus dem eingesetzten Massestrom der Reaktionsmischung aus Verfahrensschritt a) geteilt durch das eingesetzte Schüttvolumen des Katalysators. Der Wasserstoffstrom wird über einen Massemesser vor Reaktoreintritt und über die Abgasmenge nach einem dem Reaktor nachgeschalteten Hochdruckphasentrenner gemessen und reguliert.

Die Zusammensetzung der Reaktionsmischung aus dem Verfahrensschritt a) entsprach im Mittel der "rohen" Zusammensetzung des Batchversuches (s.o.). Die Vorlage der Reaktionsmischung ist auf 50°C geheizt und gerührt, um ein Ausstocken bzw. eine Heterogenisierung zu verhindern. Zur Differenzierung der Systemleistungsfähigkeit wurden die Versuche bei unterschiedlichen Katalysatorbelastungen und Temperaturen durchgeführt.

### II.1 Mechanische Stabilität

Die unterschiedlichen Katalysatoren wurden als Funktion der LHSV bei einer Temperatur von 135°C einer kontinuierlichen Hydrierung über 20 Tage unterzogen.

| | **Menge Metall in ppm im Produkt** | **Kommentar** |
|---|---|---|
| Raney^{™}-Cobalt | 0,06 | - |
| Raney^{™}-Kupfer | 0,12 | Katalysatorpartikel lösen sich. Schlechte mechanische Stabilität |
| Raney^{™}-Nickel | 0,01 | - |
| Nickel | 6-7 (Silizium) | Träger löst sich |

### II.2 LHSV-Abhängigkeit

Die Abhängigkeit der Selektivität und des Umsatzes wurden bei T=135°C und verschiedenen Durchsätzen (LHSV 0,35 und 1,00 h⁻¹) über je 5 Tage verfolgt. Angegeben ist der Mittelwert aus 5 Einzelmessungen. Es wurden folgende Ergebnisse erhalten.

| **Umsatz in % bei** | **LHSV = 0,35 h⁻¹** | **LHSV = 1,00 h⁻¹** |
|---|---|---|
| Raney^{™}-Cobalt | 100,0 | 100,0 |
| Raney^{™}-Kupfer | 100,0 | 99,9 |
| Raney^{™}-Nickel | 99,8 | 79,1 |
| Nickel | 100,0 | 99,5 |

| **Selektivität bei** | **LHSV = 0,35 h⁻¹** | **LHSV = 1,00 h⁻¹** |
|---|---|---|
| Raney^{™}-Cobalt | 101,2* | 100,1** |
| Raney^{™}-Kupfer | 99,0 | 95,3 |
| Raney^{™}-Nickel | 99,4 | 61,0 |
| Nickel | 99,0 | 98,8 |

| | | |
|---|---|---|
| *Eine rechnerische Selektivität von >100% ergibt sich durch Spaltung von Nebenkomponenten zu NPG. "bei LHSV = 1,2 h⁻¹ | | |

### II.3 Temperatur-Abhängigkeit

Die Temperaturabhängigkeit wurde für den Raney^{™}-Cobalt-Katalysator und den geträgerten Nickel-Katalysator in einem weiteren Test untersucht. Hierbei wurde ein Durchsatz von LHSV = 0,6 h⁻¹ gewählt. Bei 145°C zeigen beide Katalysatoren vollen Umsatz. Bei 95°C zeigen sich die deutlichen Unterschiede. Der Raney^{™}-Cobalt-Katalysator liefert mit 99,9% noch annähernd vollen Umsatz, während der geträgerte Nickel-Katalysator im Schnitt 90,4% Umsatz liefert.

| **Umsatz in % LHSV = 0,6 h⁻¹** | **T = 95°C** | **T = 145°C** |
|---|---|---|
| Raney^{™}-Cobalt | 99,9 | 100,0 |
| Nickel | 90,4 | 100,0 |

### II.4 Ameisensäureumsatz

Der Umsatz der Ameisensäure wurde bei 120°C und einer LHSV von 0,4 h⁻¹ untersucht. Der geträgerte Nickel-Katalysator setzt im Durchschnitt aus 54 Einzelmessungen 91,2% der im Edukt enthaltenen Ameisensäure um. Der Raney^{™}-Cobalt-Katalysator setzt hingegen 99,5% der Ameisensäure um. Bei niedrigen Temperaturen und hohen Durchsätzen sinkt der Umsatz der Ameisensäure. Bei T = 95°C und LHSV = 0,6 h⁻¹ liegt der Ameisensäure-Umsatz für den geträgerten Nickel Katalysator bei 35,6% und für den Raney^{™}-Cobalt-Katalysator bei 59,4%. Hohe Umsätze der Ameisensäure sind wünschenswert, um die Bildung von NPG-Formiaten in der Destillation zu vermeiden. Der Raney^{™}-Cobalt-Katalysator bietet im Vergleich zum geträgerten Nickel-Katalysator die Möglichkeit, die Reaktion auch bei wesentlich höheren Durchsätzen und niedrigeren Temperaturen durchzuführen.

### II.5 Umsatz - NPG-iso-Butyrat und HPN

Die Umsätze der Höhersieder NPG-mono-iso-Butyrat und HPN wurden bei T = 145°C und LHSV = 0,35 h⁻¹ untersucht. Es zeigte sich, dass der Raney^{™}-Cobalt Katalysator die besten Umsätze lieferte. Hohe Umsätze bei den Hochsiedern sind vorteilhaft, da diese in Wertprodukt gespalten werden können (NPG-Mono-iso-Butyrat liefert 1 Äquivalent und HPN 2 Äquivalente NPG). Die Versuche wurden bei T = 145°C und einer von LHSV = 0,35 h⁻¹ durchgeführt.

| **Umsatz in %** | **NPG-mono-iso-Butyrat** | **HPN** |
|---|---|---|
| Raney^{™}-Cobalt | 21,7 | 26,0 |
| Raney^{™}-Kupfer | 15,89 | 17,1 |
| Raney^{™}-Nickel | 2,1 | 9,4 |
| Nickel | 18,0 | 22,0 |

### II.6 Umsatz Tri-n-Propylamin

Um die verschiedenen Umsätze von Tri-n-Propylamin zu untersuchen, wurde in einem insgesamt 14-wöchigen Testprogramm der Raney^{™}-Cobalt Katalysator mit dem geträgerten Nickel-Katalysator verglichen. In der Versuchseinstellung mit T = 120°C und LHSV = 0,4 h⁻¹ zeigte sich bei den Durchschnittswerten aus 54 Einzelmessungen ein Umsatz von Tri-n-Propylamin von 6,99% für den geträgerten Nickel-Katalysator und 0,19% für den Raney^{™}-Cobalt-Katalysator. Bei hohen Temperaturen und niedrigen Durchsätzen (langen Verweilzeiten) steigt der Umsatz von Tri-n-Propylamin an. Bei einer T= 145°C und LHSV = 0,2 lagen die Umsätze für den geträgerten Nickel-Katalysator im Mittel (10 Einzelmessungen) bei 38,8% und für den Raney^{™}-Cobalt-Katalysator bei 12,4%. Für ein Recycling des TPAs zurück in Prozessschritt a) ist der Raney^{™}-Cobalt-Katalysator aufgrund der niedrigeren Werte für den Umsatz somit sehr vorteilhaft. Zudem können aus dem TPA Nebenkomponenten entstehen, die sich schlecht vom Wertprodukt abtrennen lassen.

## Patentansprüche

1. Verfahren zur kontinuierlichen Herstellung von C4-C10 Diolen aus C3-C9 Aldehyden umfassend die Verfahrensschritte:
a. basenkatalysierte Addition von Formaldehyd an C3-C9 Aldehyde unter Erhalt der entsprechenden Hydroxyaldehyde, und
b. anschließende Hydrierung der Hydroxyaldehyde zu den entsprechenden Diolen,
**dadurch gekennzeichnet,**
**dass** die Hydrierung der Hydroxyaldehyde ohne Aufarbeitung der Reaktionsmischung aus dem Verfahrensschritt a) kontinuierlich in flüssiger Phase an einem Raney-Cobalt-Katalysator in Gegenwart von Wasserstoff durchgeführt wird.

2. Verfahren nach Anspruch 1, wobei der Raney-Cobalt-Katalysator in der Form eines Festbettes vorliegt.

3. Verfahren nach einem der vorhergehenden Ansprüche, wobei im Verfahrensschritt a) Iso-Butanal mit wässriger Formaldehydlösung zu Hydroxypivalaldehyd umgesetzt und im Verfahrensschritt b) das Hydroxypivalaldehyd zu Neopentylglykol hydriert wird.

4. Verfahren nach einem der vorhergehenden Ansprüche, wobei die Base im Verfahrensschritt a) ausgesucht ist aus der Gruppe bestehend aus Trimethylamin, Triethylamin, Tri-n-propylamin oder Tri-n-butylamin oder Mischungen mindestens zweier Bestandteile daraus.

5. Verfahren nach einem der vorhergehenden Ansprüche, wobei der Raney-Cobalt-Katalysator weitere Metalle ausgewählt aus der Gruppe bestehend aus Chrom, Molybdän, Eisen, Nickel, Kupfer, Ruthenium, Palladium, Platin, Mangan, Rhodium oder Mischungen mindestens zweier Bestandteile daraus umfasst.

6. Verfahren nach einem der vorhergehenden Ansprüche, wobei der Raney-Cobalt-Katalysator neben Cobalt auch die Metalle Chrom und Nickel in einem Gewichtsanteil von größer oder gleich 0,05% und kleiner oder gleich 10% umfasst.

7. Verfahren nach einem der vorhergehenden Ansprüche, wobei die Hydrierung im Verfahrensschritt b) bei einer Temperatur von größer oder gleich 70°C und kleiner oder gleich 160°C durchgeführt wird.

8. Verfahren nach einem der vorhergehenden Ansprüche, wobei die Katalysatorbelastung LHSV (liquid hourly space velocity) im Verfahrensschritt b) größer oder gleich 0,3 h⁻¹ und kleiner oder gleich 1,5 h⁻¹ beträgt.

9. Verfahren nach einem der vorhergehenden Ansprüche, wobei die Konzentration der Base im Verfahrensschritt b) größer oder gleich 3 Gew.-% und kleiner oder gleich 15 Gew.-% beträgt.

10. Verfahren nach einem der vorhergehenden Ansprüche, wobei das molare Verhältnis von Wasserstoff zu Hydroxyaldehyd im Verfahrensschritt b) größer oder gleich 1:1 und kleiner oder gleich 100:1 beträgt.

11. Verfahren nach einem der vorhergehenden Ansprüche, wobei der Druck im Verfahrensschritt b) größer oder gleich 60 MPa und kleiner oder gleich 120 MPa beträgt.

12. Verfahren nach einem der vorhergehenden Ansprüche, wobei der Hydroxyaldehydgehalt zu Beginn des Verfahrensschritts b) größer oder gleich 15 Gew.-% und kleiner oder gleich 85 Gew.-% beträgt.

13. Verfahren nach einem der vorhergehenden Ansprüche, wobei die flüssige Phase im Verfahrensschritt b) einen Wassergehalt von größer oder gleich 5 Gew.-% und kleiner oder gleich 70 Gew.-% aufweist.

14. Verfahren nach einem der vorhergehenden Ansprüche, wobei die Reaktionslösung in Verfahrensschritt b) Ester umfasst, wobei der Estergehalt zu Beginn des Verfahrensschrittes b) größer oder gleich 0 Gew.-% und kleiner oder gleich 20 Gew.-% beträgt.

15. Verfahren nach einem der vorhergehenden Ansprüche, wobei die Reaktionslösung in Verfahrensschritt b) Ameisensäure, deren Salze und/oder Ameisensäure-Amin-Addukte umfasst, wobei deren Gehalt zu Beginn des Verfahrensschrittes b) größer oder gleich 0 Gew.-% und kleiner oder gleich 3 Gew.-% beträgt.

## Claims

1. Process for continuous production of C4-C10 diols from C3-C9 aldehydes comprising the process steps of:
a. base-catalyzed addition of formaldehyde onto C3-C9 aldehydes to obtain the corresponding hydroxyaldehydes and
b. subsequent hydrogenation of the hydroxyaldehydes to afford the corresponding diols,
**characterized**
**in that** the hydrogenation of the hydroxyaldehydes is performed continuously in the liquid phase over a Raney cobalt catalyst in the presence of hydrogen without workup of the reaction mixture from the process step a).

2. Process according to Claim 1, wherein the Raney cobalt catalyst is in the form of a fixed bed.

3. Process according to any one of the preceding claims, wherein isobutanal is reacted with aqueous formaldehyde solution to afford hydroxypivalaldehyde in the process step a) and the hydroxypivalaldehyde is hydrogenated to afford neopentyl glycol in the process step b).

4. Process according to any one of the preceding claims, wherein the base in the process step a) is selected from the group consisting of trimethylamine, triethylamine, tri-n-propylamine or tri-n-butylamine or mixtures of at least two constituents thereof.

5. Process according to any one of the preceding claims, wherein the Raney cobalt catalyst comprises further metals selected from the group consisting of chromium, molybdenum, iron, nickel, copper, ruthenium, palladium, platinum, manganese, rhodium or mixtures of at least two constituents thereof.

6. Process according to any one of the preceding claims, wherein the Raney cobalt catalyst contains not only cobalt but also the metals chromium and nickel in a weight fraction of greater than or equal to 0.05% and not more than or equal to 10%.

7. Process according to any one of the preceding claims, wherein the hydrogenation in the process step b) is performed at a temperature of greater than or equal to 70°C and not more than or equal to 160°C.

8. Process according to any one of the preceding claims, wherein the catalyst loading LHSV (liquid hourly space velocity) in the process step b) is greater than or equal to 0.3 h⁻¹ and not more than or equal to 1.5 h-¹.

9. Process according to any one of the preceding claims, wherein the concentration of the base in the process step b) is greater than or equal to 3% by weight and not more than or equal to 15% by weight.

10. Process according to any one of the preceding claims, wherein the molar ratio of hydrogen to hydroxyaldehyde in the process step b) is greater than or equal to 1:1 and not more than or equal to 100:1.

11. Process according to any one of the preceding claims, wherein the pressure in the process step b) is greater than or equal to 60 MPa and not more than or equal to 120 MPa.

12. Process according to any one of the preceding claims, wherein the hydroxyaldehyde content at the beginning of the process step b) is greater than or equal to 15% by weight and not more than or equal to 85% by weight.

13. Process according to any one of the preceding claims, wherein the liquid phase in the process step b) comprises a water content of greater than or equal to 5% by weight and not more than or equal to 70% by weight.

14. Process according to any one of the preceding claims, wherein the reaction solution in process step b) comprises esters, wherein the ester content at the beginning of the process step b) is greater than or equal to 0% by weight and not more than or equal to 20% by weight.

15. Process according to any one of the preceding claims, wherein the reaction solution in process step b) comprises formic acid, salts thereof and/or formic acid-amine adducts, wherein the content thereof at the beginning of the process step b) is greater than or equal to 0% by weight and not more than or equal to 3% by weight.

## Revendications

1. Procédé de fabrication continue de diols en C4 à C10 à partir d'aldéhydes en C3 à C9, comprenant les étapes de procédé :
a. addition catalysée par des bases de formaldéhyde sur des aldéhydes en C3 à C9, moyennant l'obtention des hydro-aldéhydes correspondants, et
b. hydratation ultérieure des hydro-aldéhydes en diols correspondants,
**caractérisé en ce**
**que** l'hydratation des hydro-aldéhydes est effectuée sans traitement du mélange réactionnel provenant de l'étape de procédé a) en continu sous phase liquide sur un catalyseur de cobalt de Raney en présence d'hydrogène.

2. Procédé selon la revendication 1, dans lequel le catalyseur de cobalt de Raney est présent sous la forme d'un lit solide.

3. Procédé selon l'une des revendications précédentes, dans lequel de l'iso butanal est converti avec une solution de formaldéhyde en hydroxy pivalaldéhyde dans l'étape de procédé a) et l'hydroxy pivalaldéhyde est hydraté en néopentyl glycol dans l'étape de procédé b).

4. Procédé selon l'une des revendications précédentes, dans lequel la base dans l'étape de procédé a) est choisie dans le groupe constitué de la triméthyl amine, de la triéthyl amine, de la tri-n-propyl amine ou de la tri-n-butyl amine ou de mélanges d'au moins deux composants parmi elles.

5. Procédé selon l'une des revendications précédentes, dans lequel le catalyseur de cobalt de Raney comprend d'autres métaux choisis dans le groupe constitué du chrome, du molybdène, du fer, du nickel, du cuivre, du ruthénium, du palladium, du platine, du manganèse, du rhodium ou de mélanges d'au moins deux composants parmi eux.

6. Procédé selon l'une des revendications précédentes, dans lequel le catalyseur de cobalt de Raney comprend, à côté du cobalt, également des métaux chrome et nickel dans une proportion en poids égale ou supérieure à 0,05 % et inférieure ou égale à 10 %.

7. Procédé selon l'une des revendications précédentes, dans lequel l'hydratation dans l'étape de procédé b) est effectuée à une température égale ou supérieure à 70 °C et inférieure ou égale à 160 °C.

8. Procédé selon l'une des revendications précédentes, dans lequel la charge catalytique LHSV (liquid hourly space velocity, vitesse spatiale horaire de liquide), dans l'étape de procédé b) est égale ou supérieure à 0,3 h⁻¹ et inférieure ou égale à 1,5 h⁻¹.

9. Procédé selon l'une des revendications précédentes, dans lequel la concentration de la base dans l'étape de procédé b) est égale ou supérieure à 3 % en poids et inférieur ou égale à 15 % en poids.

10. Procédé selon l'une des revendications précédentes, dans lequel le ratio molaire de l'hydrogène sur l'hydroxy aldéhyde dans l'étape de procédé b) est égal ou supérieur à 1 : 1 et inférieur ou égal à 100 : 1.

11. Procédé selon l'une des revendications précédentes, dans lequel la pression dans l'étape de procédé b) est égale ou supérieure à 60 MPa et inférieure ou égale à 120 MPa.

12. Procédé selon l'une des revendications précédentes, dans lequel la teneur en hydroxy aldéhyde au début de l'étape de procédé b) est égale ou supérieure à 15 % en poids et inférieure ou égale à 85 % en poids.

13. Procédé selon l'une des revendications précédentes, dans lequel la phase liquide dans l'étape de procédé b) présente une teneur en eau égale ou supérieure à 5 % en poids et inférieure ou égale à 70 % en poids.

14. Procédé selon l'une des revendications précédentes, dans lequel la solution réactionnelle dans l'étape de procédé b) comprend un ester, où la teneur en ester au début de l'étape de procédé b) est égale ou supérieure à 0 % en poids et égale ou inférieure à 20 % en poids.

15. Procédé selon l'une des revendications précédentes, dans lequel la solution réactionnelle dans l'étape de procédé b) comprend de l'acide formique, ses sels et/ou des produits d'addition d'acide formique et d'amine, où leur teneur au début de l'étape de procédé b) est égale ou supérieure à 0 % en poids et égale ou inférieure à 3 % en poids.
